# EUROPEAN PATENT APPLICATION

(11) **EP 3 398 523 A1**
(43) Date of publication of application: **07.11.2018**
(21) Application number: 16881137.0
(22) Date of filing: 26.12.2016
(51) Int. Cl.: A61B 17/02

(54) **LEFT ATRIAL APPENDAGE CLOSURE DEVICE**

(30) Priority: 29.12.2015 CN 201511003714; 02.03.2016 CN 201610119169
(71) Applicant: Shenzhen KYD Biomedical Technology Co. Ltd, Pingshan District Shenzhen Guangdong 518118 (CN)
(72) Inventor: CHEN, YiLong, Shenzhen, Guangdong 518118 (CN); HUANG, Wei, Shenzhen, Guangdong 518118 (CN)
(74) Representative: FRKelly
(86) International application number: PCT/CN2016/112062
(87) International publication number: WO 2017/114348

(57) **Abstract**

A left atrial appendage closure apparatus for obstructing the left atrial appendage, comprises a sealing plate and an anchored plate. The sealing plate is in a mesh structure and arranged with a choke membrane inside, in addition, the sealing plate adopts a tubular member for the distal fixation and is connected with the anchor plate by the tubular member. The proximal end of the sealing plate is fixed with a fastener provided with a structure connecting a convey device, meanwhile, the end of the tubular member at the other side away from the end fixed the sealing plate is arranged with a plurality of supporting rods, which are intersecting radially arranged to form the anchored plate.

## Description

### TECHNICAL FIELD

The present invention relates to an implantable medical device and in particular relates to a highly effective, repeatable deployment, stable fixed and fatigue resistant left atrial appendage closure apparatus which enables to closure the left atrial appendage and obstruct the outflow of thrombus in the left atrial appendage.

### BACKGROUND ART

At present, with the development of the interventional apparatus, many untreatable diseases with surgery have been treated. Compared with surgical treatment, the interventional therapy possesses the advantages of less trauma, faster recovery and better efficacy. During the past half century, the interventional therapy has been developed rapidly, and various kinds of interventional medical devices have also been created to be used for treating more and more diseases and patients.

Atrial fibrillation (AF) is one of the most common arrhythmias. Apart from the discomfort brought by the symptom of palpitation, the occurrence of the thromboembolism and other complications are the main hazards of atrial fibrillation. Among the patients with non-valvular and non-rheumatic atrial fibrillation, about 90% of the thrombus originated from the left atrial appendage. Every year, the thromboembolic events and stroke occur in approximately 5% patients with atrial fibrillation. The fatality rate caused by stroke is up to 50%. Therefore, it is of important clinical significance to know how to prevent and treat the stroke caused by atrial fibrillation thromboembolism. At present, the prevention and treatment methods of atrial fibrillation thrombus include surgeries, drug treatment and interventional therapy. Among all the methods, the occlusion of left atrial appendage by surgical procedure has not been widely used because of its great trauma and high risk. At present, the most common means to prevent stroke in patients with atrial fibrillation is receiving long-term oral anticoagulant drugs, but the anticoagulant drugs will induce the bleeding risk and about 14% to 44% of patients fail to receive long-term anticoagulant therapy because of contraindications. In recent years, a most advanced interventional therapy has been developed at home and abroad. Through a tiny catheter, the tailor-made left atrial appendage closure apparatus is implanted into the left atrial appendage to closure the left atrial appendage and to prevent and treat thrombus events and stroke in patients with atrial fibrillation. This therapy has become the first choice for the prevention and treatment of stroke caused by atrial fibrillation with the advantages of small trauma, low risk, quick recovery and good efficacy. Currently, this kind of instrument can be categorized into braiding, cutting and hybrid types from the view of structure. These types of left atrial appendage closure apparatus can all be inserted into the left atrial appendage through catheter intervention to achieve the occlusion effect of the left atrial appendage, but considerable limitations are existing among all of them. The left atrial appendages are complex in anatomical structures and different in shape from person to person, including the elliptical and peanut shapes, etc. The inner wall of the left atrial appendage is uneven, the shape is irregular and the wall is thin, so it is difficult to realize both the non piercement of the wall and the solid fixation inside the left atrial appendage without falling off, and ultimately achieve the ideal occlusion effect.

For example, the braided-type left atrial appendage closure apparatus related to the invention of patents CN1799521A and CN101146570A. They anchored the closure apparatus by using the uneven structure on the surface of the fixation plate of closure apparatus and applying the sealing plate at the opening of the left atrial appendage to achieve the effect of occlusion. The advantages of this design are simple in structure, less difficulty in processing and low in cost. But the lower anchorage strength of the braided fixation plate makes it difficult to adapt to the different shapes of the left atrial appendage. Even if the anchor hook is sutured on the fixation plate, the fixation plate is also difficult to stab into the left atrial appendage wall under the condition of the extrusion deformation to achieve the stable fixation. Moreover, the shape of the sealing plate will be affected by the extrusion deformation of fixed plate in the left atrial appendage, which leads to the poor sealing effect.

For example, the cutting-type left atrial appendage closure apparatus related to the invention of patents CN1711978A and CN1342056A. The nickel titanium metal tubes are cut and formed the designed shape by thermal treatment and processing. An outward anchor pin is designed in the part in contact with the inner wall of the left atrial appendage to stab into the inner wall of the left atrial appendage to achieve stable fixation. Generally speaking, the choke membrane is usually sutured on the cut-type mesh frame to achieve the effect of left atrial appendage occlusion. The advantage of this structure design is that the whole closure apparatus is integrated and rigid, which makes it easy to be fixed inside the left atrial appendage. The anchor pins designed are easy to pierce into the inner wall of the left atrial appendage for stable fixation and prevention of closure apparatus from falling off. However, the cutting type of left atrial appendage closure apparatus is limited in the deformation capacity and fails to adapt to left atrial appendages with various shapes, which limit its application to a great extent. After the cutting-type left atrial appendage closure apparatus is inserted into the left atrial appendage, the part in the left atrial appendage will usually form a depression with the atrial wall to form a new "left atrial appendage area", which will increase the risk of new thrombosis. The anchor pins of the cut-type left atrial appendage closure apparatus are usually stabbed to the left atrium to stabilize the closure apparatus and prevent the closure apparatus from falling off. But the anchor pins stabbed to the left atrium will be stuck to the edge of the sheath wall during the repeated positioning and the closure apparatus recovery to induce the failure of closure apparatus recovery and repeated positioning.

For example, the hybrid-type left atrial appendage closure apparatus related to the invention of patents CN102805654A and CN103099652A. In these designs, the sealing plate adopts a braided type and the choke membrane is usually sutured in the sealing plate to closure the left atrial appendage. The fixation plate is made by cutting nickel titanium metal tubes and forms into the designed shape by thermal treatment and processing. In the part in contact with the inner wall of the left atrial appendage, there are outwards anchor pins stabbing into the inner wall of the left auricle to achieve stable fixation. The hybrid-type left atrial appendage closure apparatus effectively solves most of the problems existing in the cut type of left atrial appendage closure apparatus. However, the sealing plate related to CN102805654A possesses a plane structure, which is not suitable for the sealing in arc structure of the left atrium; in addition, the supporting rods of the anchor plate connect with each other and the deformation of the joint parts is large, which will induce large processing difficulty, poor fatigue resistant performance, brittle in human body and further resulting in the risk of cardiac perforation. In addition, an anchor pin in the fixation plate of CN103099652A usually spines to the left atrium to stabilize the closure apparatus and prevent the closure apparatus from falling off. But the anchor pins stabbing into the left atrium will be stuck to the edge of the sheath wall in the process of repeat positioning and the closure apparatus recovery, which leads to the failure of repeat positioning and the closure apparatus recovery. Moreover, the fixation plate of the hybrid-type left atrial appendage closure apparatus usually has large deformation to reverse the direction of the anchor pin, which results in the tension and the shape variable of the fixation plate to reach the extreme of the material. This instrument has poor fatigue strength in permanent implantation, is easy to break and pierce the heart and other tissues to further threaten the life of the patients.

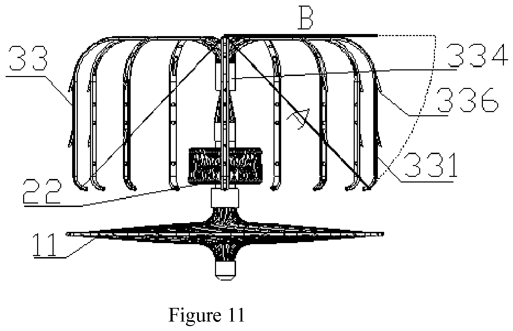

Moreover, the CN104352261A discloses an improved structure of the left atrial appendage closure apparatus, in which the connecting plate provides supporting for the anchor plate to form a rigid structure to enlarge the deformation and increase the processing difficulty. In addition, the plane structure of the anchor plate is larger than the diameter of the anchor plate in the processes of unfolding and recovery, which makes it easy to pierce the heart and other tissues and threaten the life. Figure 11 is a diagrammatic sketch of the structure in opening status.

Therefore, considering the advantages and disadvantages of all kinds of left atrial appendage closure apparatus, it is very urgent to develop a left atrial appendage closure apparatus, which can effectively closure the left atrial appendage, repeatable deployment, stably fixed and permanently implanted.

### BRIEF SUMMARY OF THE INVENTION

Some embodiments aim to provide an effective, repositionable, stably fixed and fatigue resistant left atrial appendage closure apparatus for closuring the left atrial appendage (LAA) and blocking the outflow of thrombus in the left atrial appendage.

For this purpose, the embodiments of the present invention provide a technical scheme for providing a left atrial appendage closure apparatus, comprising a sealing plate and an anchor plate connected to the sealing plate.

The sealing plate is in a mesh structure and arranged with a choke membrane inside, the sealing plate's distal end is fixed by a tubular member and is connected with the anchor plate by the other end of the tubular member which is opposite to the end fixed the sealing plate, the proximal end of the sealing plate is fixed with a fastener and has a structure can connect a convey device.

The end of the tubular member at the other side away from the end fixed the sealing plate is arranged with a plurality of supporting rods, the supporting rods originate from the direction away the sealing plate and the proximal of the supporting rods cross the center of the tubular member to the opposite side, the supporting rods are intersecting radially arranged to form the anchored plate, the angle α between the center axis of the tubular member and the proximal ends of the supporting rods is 40-55° and the distal ends of the supporting rods are in an inwardly curved shape.

On the condition that the sealing plate and anchor plate are unfolding, the height of the whole closure apparatus is 12-20 millimeters, the height of the anchor plate is 9-15 millimeters, and the height of the sealing plate is 3-5 millimeters.

According to the aforementioned preferred structure design, the anchor plate of the left atrial appendage closure apparatus of the embodiment is in a depression shape, which helps to launch and recover the anchor plate with the sheath tube of the convey device. When the sheath tube launches or recovers the closure apparatus, the anchor plate in depression shape in the shrinking process has a diameter not larger than that in the expansion process, which enables the anchor plate to slowly shrink into the sheath tube of the convey device so as to avoid the damage of left atrial appendage and improve the safety efficiency.

Preferably, the length of the distal inward bending part of the supporting rods is 1-5 millimeters. The number of the supporting rods is natural number selected from 2 to 50, which is preferred as an odd number. The inward bending part of the distal end of at least one supporting rod has more than one anchor pin on the side near the auricle. The extendable angle between the anchor pin and the supporting rod is 20-45 degrees. On the condition that the anchor plate is unfolding, the anchor plate is capable of against the inner wall of the left atrial appendage, and the anchor pin is capable of stabbing into the inner wall of the left atrial appendage to stabilize the fixation of the anchor plate in the left atrial appendage, which achieves a good fixation effect of the anchor plate made of the supporting rods in the inner of the left atrial appendage.

The distal end of the supporting rod is provided with a round ball head, the proximal end of the sealing plate is fixed with the fastener and the structure of the fastener for connecting with the convey device is a screw thread structure. The round ball head may prevent the end of the supporting rod from damaging the left atrial during expansion or recovering, the arranged screw thread structure is beneficial to the transmission, expansion and recovery of the left auricle closure apparatus.

The sealing plate is little larger than the anchor plate in diameter, in which, on the condition that the sealing plate is 2-6mm larger than the anchor plate in diameter, it is suitable for single lobe left atrial appendage, and on the condition that the sealing plate is 6-12mm larger than the anchor plate in diameter, it is more suitable for the dual-lobe left atrial appendage. The closure apparatus could fit the structure of the left atrial appendage. Therefore, the application range is more extensive.

In the perspective of processing program, the root of the anchor pin is on the supporting rod, the shape of the anchor pin is laser-cutting out and the angle of the anchor pin is finalizing by thermal treatment with the mould. The anchor pin expands automatically outwards on the condition without pressure and retracts onto the supporting rod when with pressure. The sealing plate and the anchor plate adopt the memorable alloy material, wherein the nitinol wire is preferred to form the desired shape by thermal treatment. The tubular member is selected from steel sleeves or other types of alloy sleeves.

The further preferred scheme is that the supporting rods have holes to be used to suture the PET (polyethylene terephthalate) choke membrane on the anchor plate to prevent the outflow of thrombus in the left atrial appendage.

The choke membrane is as an unmodified or chemically modified PET choke membrane.

The PET choke membrane could be a chemically modified PET choke membrane, which has an amide group by an exchange reaction with an ester group. The contact angle of the chemically modified PET choke membrane is less than 90 degrees, which enhances the hydrophilicity. The membrane surface chemical treatment of the choke membrane induces the surface negative ionization, which not only reduces the platelet adhesion on the surface of the sealing plate, but also improves the hydrophilicity and biological compatibility of the choke membrane as well as achieves rapid endothelialization. The endothelialization degree in a short period is much higher than that of the unmodified PET choke membrane, which reduces the closure apparatus associated thrombosis risks.

Specifically, the PET choke membrane makes use of the reaction to replace the ester group for the amide group to graft the molecule with sodium sulfonate group on the surface of the choke membrane, so that the PET choke membrane becomes electronegative. This kind of PET choke membrane enables to adsorb the toluidine blue dye with positive electrical charge.

The chemical modification method of the PET choke membrane is as follows: placing a PET choke membrane in a proper container, adding the mixed solvent of water and 1, 4- dioxane, adding alkane with terminal group of amino group and sulfonic group respectively, and catalyst, stirring for 2-24 hours at 50-100 °C and removing the choke membrane after the reaction.

The mentioned alkane with the sulfonic group is selected from 3- amino propane sulfonic acid, 4- amino butane sulfonic acid, 5- amidopentane sulfonic acid and 6- amino hexane sulfonic acid.

The mentioned catalyst is selected from sodium hydroxide, potassium hydroxide or ammonia water.

A more specific preferred choice: the volume ratio of water to 1, 4- dioxaneis is 5:1. After the reaction is finished, the choke membrane is removed and washed repeatedly by deionized water and absolute alcohol.

For example, a chemical modification method of a PET choke membrane is as following: placing 50 pieces of PET choke membranes in a 250mL of round bottom flask, adding 200mL of mixed solvent of water and 1, 4- dioxane with the volume ratio of 5:1, adding 0.5g of alkane with terminal group of amino group and sulfonic group respectively, and Ig of catalyst, stirring for 2-24 hours at 50-100 °C, removing the choke membrane after reaction, and washing 5 times repeatedly by deionized water and anhydrous alcohol.

Compared with the existing technology, the embodiments of the present invention possess the following advantages:
The anchor plate of the left atrial appendage closure apparatus according to the invention is in a depression shape, which helps the sheath tube of the convey device to launch and recover the anchored plate. When the closure apparatus is launched or recovered by the sheath tube, the anchor plate in the depression shape has the smaller diameter in the shrinking process than in the expansion process, which enables the anchor plate to slowly shrink into the sheath tube of the convey device so as to avoid the damage of left atrial appendage and improve the safety efficiency.

The anchor plate of the left atrial appendage closure apparatus according to the invention is mild in structure deformation, stable, and is of excellent fatigue resistance performance for permanent implantation and adaptable to the left atrial appendage cavity with various shapes and sizes.

The two-stage design of the left atrial appendage closure apparatus according to the present invention enables the closure apparatus to adapt to various forms and dimensions of the left atrial appendage.

The left atrial appendage closure apparatus according to the invention has the advantages of stable anchorage and effective occlusion of left atrial appendage.

The left atrial appendage closure apparatus according to the invention is applicable to not only closure the single lobe left atrial appendage, but also closure the dual-lobe left atrial appendage.

The left atrial appendage closure apparatus according to the invention is repositionable . In some cases, if the placement is not good enough, the closure apparatus can be recovered to the sheath tube, and repositioned on the conditions without removing from a pushing rod until the satisfactory anchoring and occlusion effects are achieved, which greatly reduces the surgery risks.

The left atrial appendage closure apparatus according to the invention can use a small conveying system to further reduce the damage to the blood vessel of the convey devices in the surgery process.

The left atrial appendage closure apparatus according to the invention is flexibly connected with the pushing rod, which greatly reduces the tension exerted by the convey device such as the pushing rod to the closure apparatus and makes the placement of the closure apparatus more accurate and precise.

The choke membrane of the left atrial appendage closure apparatus according to the invention is chemically treated to generate the negative surface ionization, which not only reduces the platelet adhesion on the surface of the sealing plate, but also improve the hydrophilicity and biological compatibility of the choke membrane as well as achieves rapid endothelialization. The endothelialization degree in a short period of time is much higher than that of the unmodified PET choke membrane, which reduces the closure apparatus associated thrombosis risks.

The present invention will become clearer with the following description in combination with the accompanying drawings, which are used to explain the embodiments of the invention.

### BRIEF DESCRIPTION OF THE SEVERAL VIEWS OF THE DRAWINGS

Figure 1 is a diagrammatic sketch of a preferred embodiment of the left atrial appendage closure apparatus according to the invention;
Figure 2 is a diagrammatic sketch of the third embodiment of the left atrial appendage closure apparatus according to the invention;
Figure 3 is a diagrammatic sketch of the fourth embodiment of the left atrial appendage closure apparatus according to the invention;

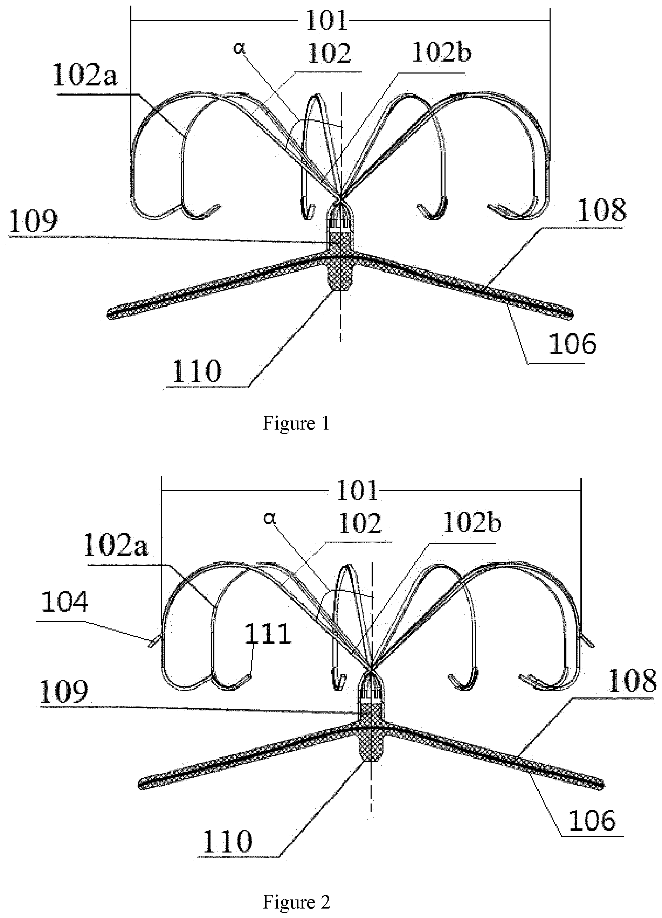

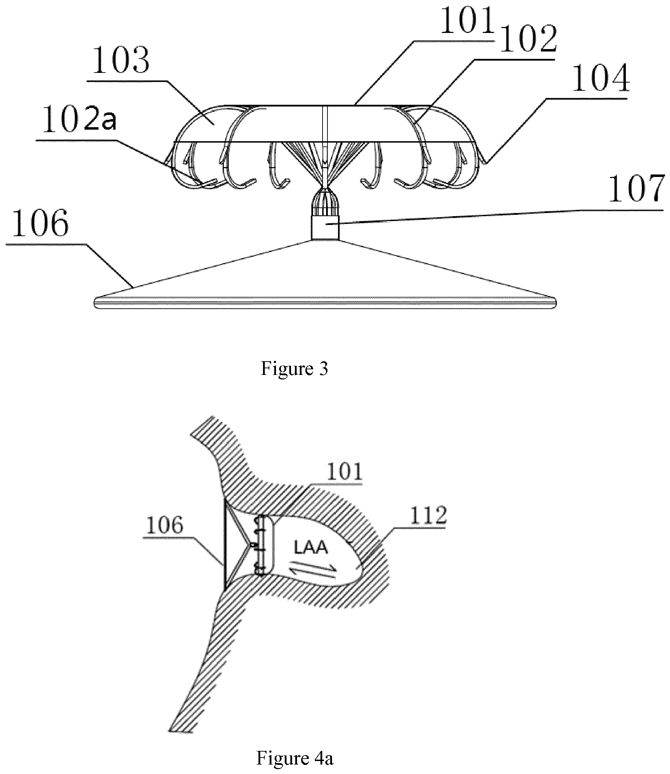

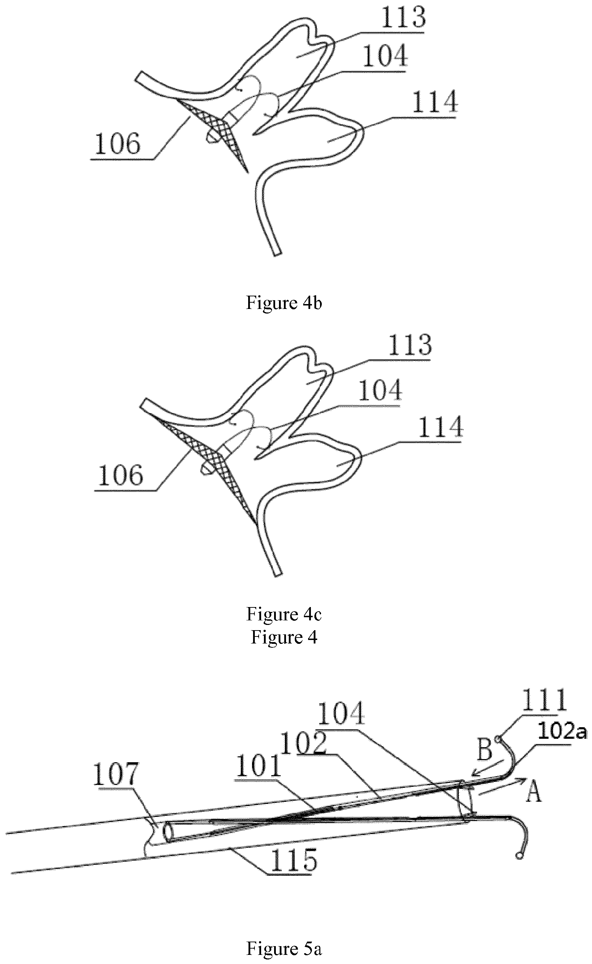

In Figure 4, Figure 4a is a position diagrammatic sketch of the fifth embodiment of the left atrial appendage closure apparatus displayed in Figure 1 in the anatomical structure of the single lobe left atrial appendage; Figure 4b is a position diagrammatic sketch of the fifth embodiment of the left atrial appendage closure apparatus displayed in Figure 1 in the anatomical structure of the dual-lobe left atrial appendage; Figure 4c is a position diagrammatic sketch of the fifth embodiment of the left atrial appendage closure apparatus displayed in figure 3 in the anatomical structure of the dual-lobe left atrial appendage;

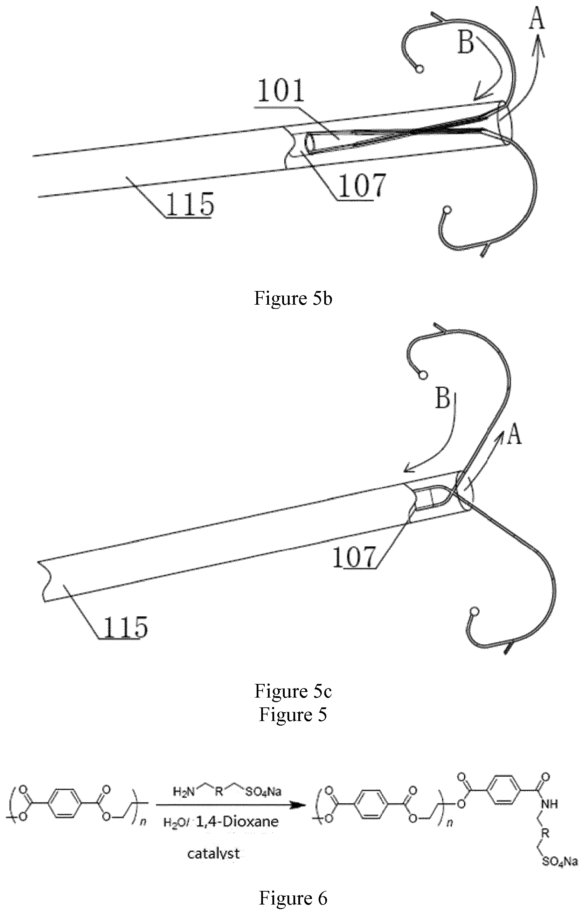

In Figure 5, Figure 5a is a schematic diagram of the status that the anchor plate of the left atrial appendage closure apparatus as shown in Figure 1 is just going out of the sheath tube of the convey device; Figure 5b is a schematic diagram of the status that the anchor plate of the left atrial appendage closure apparatus as shown in Figure 1 is going out of the sheath tube of the convey device; Figure 5c is a schematic diagram of the status that the anchor plate of the left atrial appendage closure apparatus as shown in Figure 1 is completely going out of the sheath tube of the convey device;
Figure 6 is a schematic diagram of the chemical reaction equation for the chemical modification of the choke membrane in the left atrial appendage closure apparatus according to the embodiments of the present invention, in which n = 500-5000, R: CH₂, C₂H₄, C₃H₆ or C₄H₈.
Figure 7 is a dyeing experiment schematic diagram of the chemical modification of the choke membrane in the left atrial appendage closure apparatus according to the embodiments of the present invention.
Figure 8 is a schematic diagram of the contact angle test after chemical modification of the choke membrane of the left atrial appendage closure apparatus according to the embodiments of the present invention. LA is the left contact angle and RA is the right contact angle.
Figure 9 is a histogram of the contact angle test results of the ePTFE (expanded polytetrafluoroethylene) reference substance, choke membrane before chemical modification, and choke membrane of the left atrial appendage with different chemical modification time according to the embodiments of the invention.
Figure 10 is a postoperatively anatomic image. Figure 10a and Figure 10b are respectively the anatomic image 1 day and 1 month after the surgery.
Figure 11 is the unfolded or recovery schematic diagram of the closure apparatus according to CN104352261A.

### DETAILED DESCRIPTION OF SEVERAL EMBODIMENTS OF THE INVENTION

The embodiments of the invention were described with references to the accompanying drawings. The similar element symbols in the attached drawings represented the similar elements.

### Embodiment 1

Figure 1 described a left atrial appendage closure apparatus, including a sealing plate 106 and an anchor plate 101 connected to the sealing plate.

The sealing plate was in a mesh structure and arranged with a choke membrane 108 inside, the sealing plate's distal end was fixed by a tubular member 109 and was connected with the anchor plate by the other end of the tubular member which was opposite to the end fixed the sealing plate, the proximal end of the sealing plate was fixed with a fastener 110 and had a structure can connect a convey device.

The end of the tubular member at the other side away from the end fixed the sealing plate was arranged with a plurality of supporting rods 102, the supporting rods originated from the direction away the sealing plate and the proximal ends 102b of the supporting rods crossed the tubular member center to the opposite side, the supporting rods were intersecting radially arranged to form the anchor plate 101. The angle α between the center axis of the tubular member and the proximal ends 102b of the supporting rods was 40-55° and the distal ends 102a of the supporting rods were in an inwardly curved shape.

### Embodiment 2

A left atrial appendage closure apparatus described in Figure 1, including a sealing plate 106 and an anchor plate 101 connected to the sealing plate.

The sealing plate was in a mesh structure and arranged with a choke membrane 108 inside, the sealing plate's distal end was fixed by a tubular member 109 and was connected with the anchor plate by the other end of the tubular member which was opposite to the end fixed the sealing plate, the proximal end of the sealing plate was fixed with a fastener 110 and had a structure which could connect a convey device.

The end of the tubular member at the other side away from the end fixed the sealing plate was arranged with a plurality of supporting rods 102, the supporting rods originated from the direction away the sealing plate and the proximal ends 102b of the supporting rods crossed the tubular member center to the opposite side, the supporting rods were intersecting radially arranged to form the anchor plate 101. The angle α between the center axis of the tubular member and the proximal ends 102b of the supporting rods was 40-55° and the distal ends 102a of the supporting rods were in an inwardly curved shape.

When the sealing plate and the anchor plate were unfolding, the height of the whole closure apparatus was 12-20 millimeters, the height of the anchor plate was 9-15 millimeters, and the height of the sealing plate was 3-5 millimeters.

### Embodiment 3

A left atrial appendage closure apparatus described in Figure 2, including a sealing plate 106 and an anchor plate 101 connected to the sealing plate.

The sealing plate was in a mesh structure and arranged with a choke membrane 108 inside, the sealing plate's distal end was fixed by a tubular member 109 and was connected with the anchor plate by the other end of the tubular member which was opposite to the end fixed the sealing plate, the proximal end of the sealing plate was fixed with a fastener 110 and had a structure which could connect a convey device.

The end of the tubular member at the other side away from the end fixed the sealing plate was arranged with a plurality of supporting rods 102, the supporting rods originated from the direction away the sealing plate and the proximal ends 102b of the supporting rods crossed the tubular member center to the opposite side, the supporting rods were intersecting radially arranged to form the anchor plate 101. The angle α between the center axis of the tubular member and the proximal ends 102b of the supporting rods was 40-55° and the distal ends 102a of the supporting rods were in an inwardly curved shape.

The length of the inward bending part of the distal ends 102a of the supporting rods was 1-5 millimeters. The number of the supporting rods was 6. The inward bending part of the distal end of at least one supporting rod had more than one anchor pin 104 on the side near the auricle. The extendable angle between the anchor pin and the supporting rod could be 20-45 degrees. When the anchor plate was unfolding, the anchor plate was against the inner wall of the left atrial appendage, and the anchor pin stabbed into the inner wall of the left atrial appendage to stabilize the fixation of the anchor plate in the left atrial appendage.

The distal ends of the supporting rods were provided with round ball heads 111, and the proximal end of the sealing plate was fixed with the fastener, and the structure of the fastener for connecting with the convey device was a screw thread structure.

### Embodiment 4

A left atrial appendage closure apparatus described in Figure 2, including a sealing plate 106 and an anchor plate 101 connected to the sealing plate.

The sealing plate was in a mesh structure and arranged with a choke membrane 108 inside, the sealing plate's distal end was fixed by a tubular member 109 and was connected with the anchor plate by the other end of the tubular member which was opposite to the end fixed the sealing plate, the proximal end of the sealing plate was fixed with a fastener 110 and had a structure which could connect a convey device.

The end of the tubular member at the other side away from the end fixed the sealing plate was arranged with a plurality of supporting rods 102, the supporting rods originated from the direction away the sealing plate and the proximal ends 102b of the supporting rods crossed the tubular member center to the opposite side, the supporting rods were intersecting radially arranged to form the anchor plate 101. The angle α between the center axis of the tubular member and the proximal ends 102b of the supporting rods was 40-55° and the distal ends 102a of the supporting rods were in an inwardly curved shape.

The length of the inward bending part of the distal ends 102a of the supporting rods was 1-5 millimeters. The number of the supporting rods was 6. The inward bending part of the distal end of at least one supporting rod had more than one anchor pin 104 on the side near the auricle. The extendable angle between the anchor pin and the supporting rod could be 20-45 degrees. When the anchor plate was unfolding, the anchor plate was against the inner wall of the left atrial appendage, and the anchor pin stabbed into the inner wall of the left atrial appendage to stabilize the fixation of the anchor plate in the left atrial appendage.

The distal ends of the supporting rods were provided with round ball heads 111, and the proximal end of the sealing plate was fixed with the fastener, and the structure of the fastener for connecting with the convey device was a screw thread structure.

As shown in Figure 3, the supporting rods could also be provided with holes to be used to suture the PET (polyethylene terephthalate) choke membrane on the anchor plate to prevent the outflow of thrombus in the left atrial appendage.

### Embodiment 5

A left atrial appendage closure apparatus described in Figure 2, including a sealing plate 106 and an anchor plate 101 connected to the sealing plate.

The sealing plate was in a mesh structure and arranged with a choke membrane 108 inside, the sealing plate's distal end was fixed by a tubular member 109 and was connected with the anchor plate by the other end of the tubular member which was opposite to the end fixed the sealing plate, the proximal end of the sealing plate was fixed with a fastener 110 and had a structure which could connect a convey device.

The end of the tubular member at the other side away from the end fixed the sealing plate was arranged with a plurality of supporting rods 102, the supporting rods originated from the direction away the sealing plate and the proximal ends 102b of the supporting rods crossed the tubular member center to the opposite side, the supporting rods were intersecting radially arranged to form the anchor plate 101. The angle α between the center axis of the tubular member and the proximal ends 102b of the supporting rods was 40-55° and the distal ends 102a of the supporting rods were in an inwardly curved shape.

The length of the inward bending part of the distal ends 102a of the supporting rods was 1-5 millimeters. The number of the supporting rods was 6. The inward bending part of the distal end of at least one supporting rod had more than one anchor pin 104 on the side near the auricle. The extendable angle between the anchor pin and the supporting rod could be 20-45 degrees. When the anchor plate was unfolding, the anchor plate was against the inner wall of the left atrial appendage, and the anchor pin stabbed into the inner wall of the left atrial appendage to stabilize the fixation of the anchor plate in the left atrial appendage.

The distal ends of the supporting rods were provided with round ball heads 111, and the proximal end of the sealing plate was fixed with a bolt head, and the structure of the fastener for connecting with the convey device was a screw thread structure.

As shown in Figure 4a, the sealing plate was little larger than the anchor plate in diameter, in which, if the sealing plate was 2-6 mm larger than the anchor plate in diameter, the closure apparatus was suitable for single lobe left atrial appendage.

But the dimension above was difficult to completely seal the dual-lobe left atrial appendage as displayed in Figure 4b. Therefore, another preferred scheme had been shown in Figure 4c, the sealing plate was little larger than the anchor plate in diameter, in which, if the sealing plate was 6-12mm larger than the anchor plate in diameter, the closure apparatus was more suitable for the dual-lobe left atrial appendage.

### Embodiment 6

As shown in Figures 5a, 5b and 5c, the anchor plate 101 had the following characteristics in the processes of entering the sheath tube 115 of the convey device and going out from the sheath tube 115: in order to clearly demonstrate the processes for the anchor plate 101 to enter and exit the sheath tube, the number of the supporting rods 102 of the anchor plate 101 had been simplified to 2 in Figures 5a, 5b and 5c, the supporting rods 102 were straightened in the sheath tube and were in a cross shape, the anchor pins 104 were towards to oblique medial direction opposite to the central end 107 of the tubular member. The inner wall of the sheath tube would not be scratched during the pushing process in the sheath tube 115. Under the thrust of the pushing rod, the elbows of the distal ends 102a of the supporting rods were slowly pushed out of the sheath 115 (Figure 5a) and expand along the direction A. With the continuous pushing of the pushing rod, the supporting rods 102 continued to expand along the direction A as the status displayed in Figure 5b. At this point, the anchor pins 104 turned about 90 degrees to nearly perpendicular to the pushing rod. The pushing rod continued to exert the impetus and push the anchor plate 101 completely out of the sheath 115, and the supporting rods 102 continued to expand along the direction A as the status displayed in Figure 5c. At this time, the anchor plate 101 was fully unfolded, the anchor pins 104 were turned over about 90 degrees again to locate outside the anchor plate 101 and face towards the oblique lateral direction of the central end 107, to stab into the inner wall of the left atrial appendage. The closure apparatus was fixed in the left atrial appendage cavity.

The process of recovering the anchor plate 101 was the opposite of each phase of the process of pushing the anchor plate 101 outside the sheath tube 115. The anchor plate 101 was pulled into the sheath tube 115 along the direction B by the pushing rod, wherein the remarkable characteristic was that the anchor pins 104 turned towards the oblique medial direction opposite to the central end 107 from the oblique lateral direction of the central end 107 in the expansion process. This process ensured that the anchorage pins 104 did not scrape the wall of the left atrial appendage and entered the sheath tube 115 smoothly. The left atrial appendage closure apparatus in this embodiment was not only able to ensure the repositioning function of the anchor plate 101 in the left atrial appendage, but also greatly reduced the damage to the left atrial appendage wall and reduced the risk and complications of surgery.

### Embodiment 7 A Processing Scheme

As shown in Figures 1, 2 and 3, the left atrial appendage closure apparatus related to the invention included the sealing plate 106 and the anchor plate 101 connected with the sealing plate 106, wherein the sealing plate 106 was woven into a mesh structure with nitinol wires and finalized to plate shape by thermal treatment, both the ends of the sealing plate were fixed with the steel sleeve 109 and the fastener 110 respectively, one end of the sealing plate 106 was linked to the anchor plate 101 with the steel sleeve and the other end was connected with the pushing rod of the convey device by a screw thread structure. The diameter of the sealing plate 106 was about 2-6 mm larger than that of the anchor plate 101, and the inside of the sealing plate 106 was sutured with the chemically modified PET choke membrane 108. The anchor plate 101 was made up of a multiple of supporting rods 102 cut from a nitinol tube, and was treated by thermal treatment with the mould to form the shape shown in the figures. The central end 107 of the anchor plate 101 was welded with the sealing plate 106. According to the requirements of the actual mechanical performance and specifications, the number of supporting rods could be changed. The supporting rods emanated from one inner side of the circle central end 107, crossed the center of the central end 107 to the opposite side, and were bent. The supporting rods were arranged in a cross radial way. Holes could also be arranged on the supporting rods 102 to suture the PET choke membrane on the anchor plate 101 to prevent the outflow of the thrombus in the left atrial appendage. On the supporting rods 102, the shape of the anchor pins 104 was cut out by laser and the angle of the anchor pins 104 was finalizing by thermal treatment of the mould. The anchor pins 104 obliqued laterally and faced towards the sealing plate 106. The root of the anchor pins 104 was on the supporting rods 102, the anchor pins 104 opened outward automatically without pressure and were recovered to the supporting rods 102 when with pressure. The distal ends 102a of the supporting rods were curved inward. A spherical head 111 could be arranged at the distal end of the supporting rod 102 to prevent from scratching the inner auricle when the anchor plate 101 was pushed out of the sheath.

### Embodiment 8

The PET choke membrane sutured in the sealing plate of the left atrial appendage closure apparatus according to the embodiment was a chemically modified choke membrane. As shown in Figure 6, the PET choke membrane took advantages of the reaction to exchange the ester group for an amide group to graft the molecule with sodium sulfonate group on one side onto the surface of the choke membrane, and the specific operation was as follows:
50 pieces (50×50mm²) of PET choke membrane were placed in a 250mL of round bottom flask, 200mL of mixed solvent of water and 1, 4-dioxane with the volume ratio of 5:1 were added, 0.5g of alkane with terminal group of amino group and sulfonic group, such as 3-aminopropanesulfonic acid, 4-aminobutanesulfonic acid, 5-aminopentanesulfonic acid or 6-aminohexane sulfonic acid was added respectively, then 1g catalyst, such as sodium hydroxide, potassium hydroxide or ammonia water was add. The mixture was stirred for 2-24 hours at 50-100 °C, the choke membrane was removed after the reaction, and the resulting solution was washed 5 times repeatedly by deionized water and anhydrous alcohol.

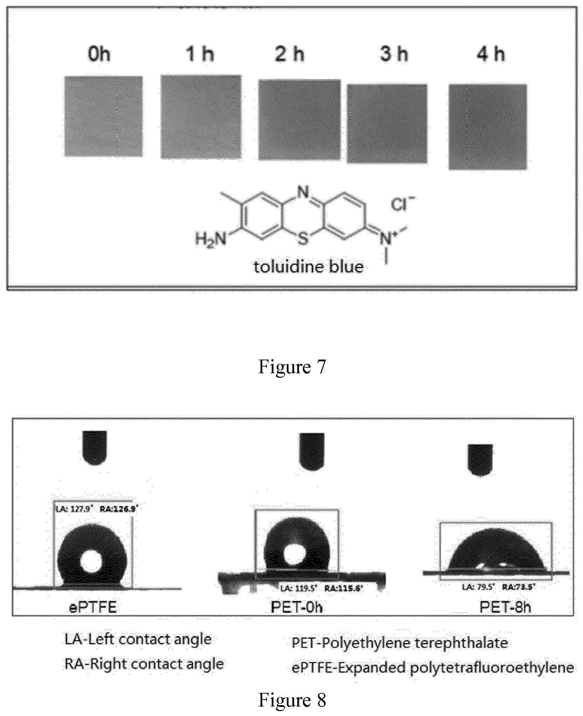

As shown in Figure 7, the PET choke membrane after the chemical reaction was immersed in the toluidine blue dye with positive electrical charge and removed immediately. By many experiments, it was found that the PET choke membrane without chemical modification did not adsorb the toluidine blue dye with positive electrical charge and the adsorbed quantity of the toluidine blue dye also gradually increased with the gradual increasing of the chemical modification time of the PET choke membrane.

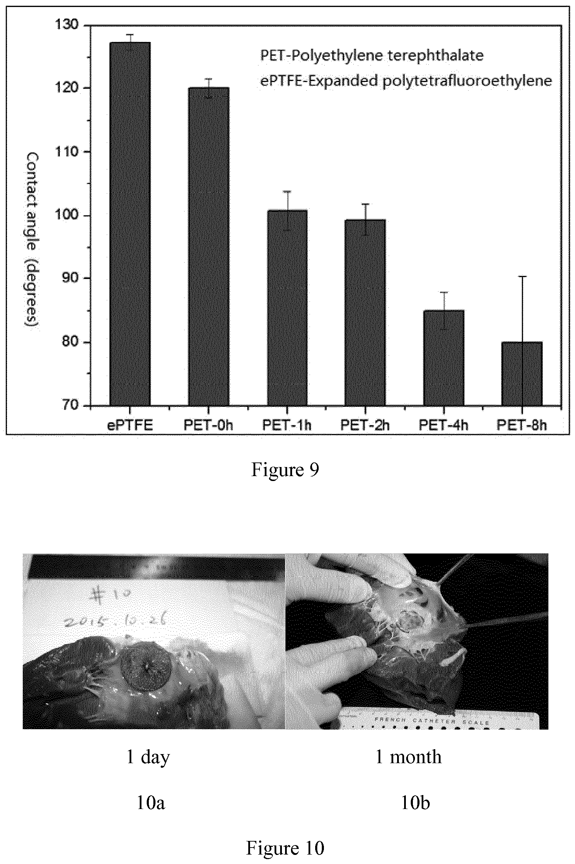

According to the contact angle test results shown in Figures 8 and 9, the contact angle between the chemically modified PET choke membrane and the water droplet was 76.5±3 degrees (as displayed in PET-8h histogram), which was 41 degrees smaller than that of the unmodified PET choke membrane, and 51 smaller than that of the super hydrophobic ePTFE film. According to the test results, the hydrophilicity of the modified PET film was greatly enhanced, which was beneficial to improve the hydrophilicity and biocompatibility of the choke membrane, to achieve rapid endothelialization and to reduce the risk of thrombosis associated with the closure apparatus.

### Embodiment 9. Animal Experiment

The closure apparatus with the chemical modified PET choke membrane in the embodiment according to the invention was adopted and the experimental method was as follows:
All the experimental dogs received percutaneous left atrial appendage occlusion under general anesthesia. They started fasting 12h before the surgery and were tied up on the operating table after anesthesia, the tracheal intubation was conducted to connect the respirator for mechanical ventilation with indwelling transesophageal ultrasound probe. The electrocardiogram monitoring was connected and venous access in the left lower extremities was established. If the anatomic abnormalities of the heart were observed in the transesophageal echocardiogram, the surgery would be stopped and the other experimental dog was replaced. For dogs with normal heart anatomy, the shape of their left atrial appendage was observed by transesophageal echocardiography and the diameter of left atrial appendage was measured to select the closure apparatus with a diameter about 15-35% larger than the measured diameter. The routine disinfection was performed and the sterile towels were spread. The right femoral artery and vein were punctured with the Telma puncture suit, and the 6F femoral sheath was retained. Through the femoral artery sheath, the 5F pigtail catheter was sent to the aortic sinus to mark the position of the aortic sinus to avoid atrial septal puncture into the aortic sinus. Through the femoral vein sheath, the atrial septal puncture needle (SL1, St Jude Medical, US) and the matched atrial septal puncture sheath were sent into to complete the transseptal puncture under the guidance of the transesophageal echocardiography, after successful puncture, 80-100U/kg of heparin was given, and the atrial septal puncture needle was withdrawn, the long exchange steel wire was sent to the left pulmonary vein to exchange 8-10F closure apparatus delivery sheath to the opening part of the left atrial appendage, and the pigtail catheter was sent to left atrial appendage for conducting the left atrial appendage angiography and measuring the opening diameter of the left atrial, the sealing plate of the closure apparatus with the diameter about 15-35% larger than the measurement value was selected in combination with the transesophageal echocardiography results, and the closure strategy was determined according to the lobe form of the left atrial appendage. The sealing plate selected was fixed on the head of the pushing rod by a nut, when the air in the closure apparatus and the conveying sheath was exhausted, slowly forwarded the pushing rod and pushed part of the anchor plate out of the delivery sheath before entering the opening of the left atrial appendage. The conveying sheath and the pushing rod as whole were pushed to the anchoring area of the left atrial appendage, and when their head reached the anchoring area of the opening of the left atrial appendage, fixed the pushing rod and withdrew the conveying sheath gradually, the closure apparatus deployed was observed by the transesophageal echocardiography and angiography of left atrial appendage, while the pushing and pulling test were performed to check whether the deployed of the closure apparatus was firm and stable. If the closure apparatus was in the proper position and the fixation was firm, the closure apparatus would be released. If the complete occlusion failed or the extension shape of the closure apparatus was improper, the closure apparatus should be completely recycled into the conveying sheath to try again to be expanded until the ideal sealing effect was achieved, then the closure apparatus could be released. After the surgery, the experimental dogs routinely took aspirin and were followed up regularly. The anatomical images (Figures 10a and 10b) on the 1st day and 1 month after surgery displayed that in the 1-2 months, the endothelial cells were uniformly endothelialized on the closure apparatus and there was no obvious thrombosis forming, which was significantly more effective than the existing technology in endothelialization and thrombosis inhibition.

Therefore, the embodiments according the present invention has the following advantages:
The anchor plate of the left atrial appendage closure apparatus according to the invention is in a depression shape, which helps the sheath tube of the convey device to launch and recover the anchored plate. When the closure apparatus is launched or recovered by the sheath tube, the anchor plate in the depression shape has the smaller diameter in the shrinking process than in the expansion process, which enables the anchor plate to slowly shrink into the sheath tube of the convey device so as to avoid the damage of left atrial appendage and improve the safety efficiency.

The anchor plate of the left atrial appendage closure apparatus according to the invention is mild in structure deformation, stable, and is of excellent fatigue resistance performance for permanent implantation and adaptable to the left atrial appendage cavity with various shapes and sizes.

The two - stage design of the left atrial appendage closure apparatus according to the present invention enables the closure apparatus to adapt to various forms and dimensions of the left atrial appendage.

The left atrial appendage closure apparatus according to the invention has the advantages of stable anchorage and effective occlusion of left atrial appendage.

The left atrial appendage closure apparatus according to the invention is applicable to not only closure the single lobe left atrial appendage, but also closure the dual-lobe left atrial appendage.

The left atrial appendage closure apparatus according to the invention can be repeatedly positioned. In some cases, if the placement is not good enough and without releasing the convey device, such as the pushing rod, the closure apparatus can be recovered to the sheath tube and repositioned until the satisfactory anchoring and occlusion effects are achieved, which greatly reduces the surgery risks.

The left atrial appendage closure apparatus according to the invention can use a small conveying system to further reduce the damage to the blood vessel of the convey devices in the surgery process.

The left atrial appendage closure apparatus according to the invention is flexibly connected with the pushing rod, which greatly reduces the tension exerted by the pushing rod to the closure apparatus and makes the placement of the closure apparatus more accurate and precise.

The choke membrane of the left atrial appendage closure apparatus according to the invention is chemically treated to generate the negative surface ionization, which not only reduces the platelet adhesion on the surface of the sealing plate, but also improve the hydrophilicity and biological compatibility of the choke membrane as well as achieves rapid endothelialization, which reduces the closure apparatus associated thrombosis risks.

The above mentioned is only a better embodiment of the present invention, and should not be used to restrict the invention. Any modification, equal substitution and improvement within the spirit and principles of the invention should be included in the protection scope of the invention.

## Claims

1. A left atrial appendage closure apparatus, comprising:
a sealing plate and an anchor plate connected to the sealing plate,
the sealing plate is in a mesh structure and arranged with a choke membrane inside, the sealing plate's distal end is fixed by a tubular member and is connected with the anchor plate by the other end of the tubular member which is opposite to the end fixed the sealing plate, the proximal end of the sealing plate is fixed with a fastener and has a structure can connect a convey device;
the end of the tubular member at the other side away from the end fixed the sealing plate is arranged with a plurality of supporting rods, the supporting rods originate from the direction away the sealing plate and the proximal of the supporting rods cross the center of the tubular member to the opposite side, the supporting rods are intersecting radially arranged to form the anchored plate, the angle α between the center axis of the tubular member and the proximal ends of the supporting rods is 40-55° and the distal ends of the supporting rods are in an inwardly curved shape.

2. The left atrial appendage closure apparatus according to claim 1, on the condition that the sealing plate and anchor plate are unfolding, the height of the whole closure apparatus is 12-20 millimeters, the height of the anchor plate is 9-15 millimeters, and the height of the sealing plate is 3-5 millimeters.

3. The left atrial appendage closure apparatus according to claim 1, wherein the length of the distal inward bending part of the supporting rods is 1-5 millimeters.

4. The left atrial appendage closure apparatus according to claim 1, wherein the number of the supporting rods is natural number selected from 2-50, which is preferred as an odd number.

5. The left atrial appendage closure apparatus according to claim 1, wherein the distal inward bending part of at least one supporting rod has more than one anchor pin on the side near the auricle, the extendable angle between the anchor pin and the supporting rod is 20-45 degrees.

6. The left atrial appendage closure apparatus according to claim 5, on the condition that the anchor plate is unfolding, the anchor plate is capable of against the inner wall of the left atrial appendage, and the anchor pin is capable of stabbing into the inner wall of the left atrial appendage to stabilize the fixation of the anchor plate in the left atrial appendage.

7. The left atrial appendage closure apparatus according to claim 5, the root of the anchor pin is on the supporting rod, the shape of the anchor pin is laser-cutting out and the angle of the anchor pin is finalizing by thermal treatment with the mould, the anchor pin expands automatically outwards without pressure and retracts onto the supporting rod when with pressure.

8. The left atrial appendage closure apparatus according to claim 1, wherein there is a round ball head at the distal end of the supporting rod, the structure of the fastener for connecting with the convey device is a screw thread structure.

9. The left atrial appendage closure apparatus according to claim 1, the diameter of the sealing plate is little larger than that of the anchored plate, wherein the diameter of the sealing plate is 2-6 mm or 6-12 mm larger than that of the anchored plate.

10. The left atrial appendage closure apparatus according to claim 1, the sealing plate adopts the memorable alloy material, wherein the nitinol wire is preferred to form the desired shape by thermal treatment, the tubular member is selected from steel sleeves or other types of alloy sleeves.

11. The left atrial appendage closure apparatus according to claim 1, wherein the supporting rods have holes to be used to suture the choke membrane on the anchor plate to prevent the outflow of thrombus in the left atrial appendage.

12. The left atrial appendage closure apparatus according to claim 1 or 11, wherein the choke membrane is an unmodified or chemically modified PET choke membrane.

13. The left atrial appendage closure apparatus according to claim 12, wherein the PET choke membrane is a chemically modified PET choke membrane, which has an amide group by an exchange reaction with an ester group, and the contact angle of the chemically modified PET choke membrane is less than 90 degrees, which enhances the hydrophilicity.

14. The left atrial appendage closure apparatus according to claim 13, wherein the reaction graft the molecule with sodium sulfonate group onto the surface of the choke membrane, so that the surface of the PET choke membrane is electronegative.

15. The left atrial appendage closure apparatus according to claim 13, wherein the chemical modification method of PET choke membrane comprise the steps: placing a PET choke membrane in a proper container, adding the mixed solvent of water and 1, 4- dioxane, then adding alkane with terminal group of amino group and sulfonic group respectively, and catalyst, stirring for 2-24 hours at 50-100 °C and removing the choke membrane after the reaction.

16. The left atrial appendage closure apparatus according to claim 15, wherein the alkane is selected from 3-amino propane sulfonic acid, 4-amino butane sulfonic acid, 5-amidopentane sulfonic acid and 6-amino hexane sulfonic acid, the catalyst is selected from sodium hydroxide, potassium hydroxide and ammonia water.

17. The left atrial appendage closure apparatus according to claim 15, wherein the volume ratio of water to 1, 4-dioxane is 5:1, the choke membrane is removed and washed repeatedly by deionized water and absolute alcohol after the reaction is finished.
